# EUROPEAN PATENT APPLICATION

(11) **EP 1 970 096 A1**
(43) Date of publication of application: **17.09.2008**
(21) Application number: 06843191.5
(22) Date of filing: 25.12.2006
(51) Int. Cl.: A61N 1/30

(54) **IONTOPHORESIS DEVICE AND METHOD FOR PRODUCING SAME**

(30) Priority: 27.12.2005 JP 2005374156
(71) Applicant: Hisamitsu Pharmaceutical Co., Inc., Tosu-shi, Saga-ken (JP); Kyodo Printing Co., Ltd., Tokyo 112-8501 (JP)
(72) Inventor: TOKUMOTO, Seiji, Tsukuba-shi, Ibaraki 3050856 (JP); ADACHI, Hirotoshi, Tsukuba-shi, Ibaraki 3050856 (JP); FUCHITA, Yasushi, Tokyo 1128501 (JP); TAKAHASHI, Saori, Tokyo 1128501 (JP); OGAWA, Tatsuya, Tokyo1128501 (JP)
(74) Representative: Shindler, Nigel
(86) International application number: PCT/JP2006/325788
(87) International publication number: WO 2007/077797

(57) **Abstract**

To provide an easily producible mass-production type iontophoresis device having a structure that a dissolution liquid-storing container is integrated with an iontophoresis electrode, a dissolution liquid and a drug can be mixed by simple operations, and it is free from a risk of leakage of electricity. The device comprises an electrode film having an electrode layer (2) formed on a base (1), a drug-loaded member (3) arranged on the electrode-layer-forming side of the electrode film, and a dissolution liquid-storing container (5) arranged on the non-electrode-layer-forming side of the electrode film, wherein the electrode film is provided with a dissolution liquid passing hole (9), a flange (5a) of the dissolution liquid-storing container (5) is bonded to the electrode film via an aluminum lid member (7) which covers the dissolution liquid passing hole (9), and the aluminum lid member (7) is arranged within the flange outer diameter of the dissolution liquid-storing container.

## Description

### TECHNICAL FIELD

The present invention relates to an iontophoresis device used in the medical field of treatment or diagnosis.

### BACKGROUND ART

The iontophoresis is a type of method for enhancement of physical absorption of a drug and administers the drug through the skin or the mucosa by applying voltage to the skin or the mucosa and electrically causing migration of the drug.

In a case where a drug which is particularly poor in stability to water is used in the iontophoresis device, it is necessary to separate the drug and a dissolution liquid from each other at the time of storage in order to prevent the drug from being deteriorated during its storage and to mix the drug and the dissolution liquid immediately before the use for treatment. For that, it is convenient to have a structure that a dissolution liquid-storing container is integrated with the iontophoresis electrode itself which includes an electrode layer to be applied to the skin or the mucosa, and the dissolution liquid and the drug can be mixed by a simple operation.

For example, Patent Literature 1 proposes a structure that a capsule in which an electrolyte solution is encapsulated is fitted to a plaster structure for iontophoresis which is composed of an electrode layer and a drug-containing layer via an aluminum foil.

### [Patent Literature 1]

### SUMMARY OF THE INVENTION

### Problems to be solved by the Invention

The structure proposed by Patent Literature 1 can mix an electrolyte and a drug by breaking the aluminum foil but has disadvantages that there is a risk of leakage of electricity from an aluminum exposed portion when current is applied, resulting in a high risk of giving an electrical shock to a human body and it lacks safety.

A disposable iontophoresis device is questioned for its mass productivity, and its structure and production method that mass production can be made easily are needed. But, the structure proposed in Patent Literature 1 has problems that it lacks productivity, mass production is not easy and the cost increases.

The present invention has been made to overcome the above problems and has an object to provide a mass production type iontophoresis device having a structure that a dissolution liquid-storing container is integrated with an iontophoresis electrode itself and a dissolution liquid and a drug can be mixed by a simple operation, it is free from a risk of leakage of electricity and easy production is made possible, and a method for producing it.

### Means for solving the Problems

The present invention configured to remedy the above-described problems has the following structures.

(1) An iontophoresis device, comprising at least an electrode film having an electrode layer formed on a base, a drug-loaded member arranged on the electrode-layer-forming side of the electrode film, and a dissolution liquid-storing container arranged on the non-electrode-layer-forming side of the electrode film, wherein the electrode film is provided with a dissolution liquid passing hole, a flange of the dissolution liquid-storing container is bonded to the electrode film via an aluminum lid member which covers the dissolution liquid passing hole, and the aluminum lid member is arranged within the flange outer diameter of the dissolution liquid-storing container.

(2) The iontophoresis device according to (1), wherein the aluminum lid member and the electrode film are bonded via an adhesive layer.

(3) The iontophoresis device according to claim (1) or (2), wherein the surface of the aluminum lid member positioned at the dissolution liquid passing hole does not have the adhesive layer.

(4) The iontophoresis device according to any one of claims (1) to (3), wherein the aluminum lid member and the dissolution liquid-storing container are bonded via a sealant layer.

(5) A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
   forming an adhesive layer on only a part of the electrode film to which the dissolution liquid-storing container is bonded,
   forming a dissolution liquid passing hole in a region of the inner side of the electrode film where the adhesive layer is formed,
   bonding an aluminum lid member having a sealant layer on one side surface to the adhesive layer, and
   bonding the dissolution liquid-storing container via the sealant layer of the aluminum lid member, and arranging the aluminum lid member within the flange outer diameter of the dissolution liquid-storing container.

(6) A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
   forming an adhesive layer on the entire surface of the side of the electrode film to which the dissolution liquid-storing container is bonded,
   forming a dissolution liquid passing hole in the electrode film,
   laminating a coating film having an opening larger than the dissolution liquid passing hole and an aluminum lid member having a sealant layer on one side surface on the adhesive layer-formed side of the electrode film, and bonding the aluminum lid member to the adhesive layer at the opening portion, and
   bonding the dissolution liquid-storing container via the sealant layer of the aluminum lid member, and arranging the aluminum lid member within the flange outer diameter of the dissolution liquid-storing container.

(7) A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
   forming an adhesive layer on only a part of the electrode film to which the dissolution liquid-storing container is bonded,
   forming a dissolution liquid passing hole in a region of the inner side of the electrode film where the adhesive layer is formed,
   bonding an aluminum lid member within the flange outer diameter of the dissolution liquid-storing container, and
   bonding the aluminum lid member which is bonded to the dissolution liquid-storing container to the adhesive layer.

(8) A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
   forming an adhesive layer on the entire surface of the side of the electrode film to which the dissolution liquid-storing container is bonded,
   forming a dissolution liquid passing hole in the electrode film,
   bonding a coating film having an opening larger than the dissolution liquid passing hole to the adhesive layer-formed side of the electrode film,
   bonding an aluminum lid member within the flange outer diameter of the dissolution liquid-storing container, and
   bonding the aluminum lid member which is bonded to the dissolution liquid-storing container to the adhesive layer at the opening portion of the coating film.

(9) A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
   forming a dissolution liquid passing hole in the electrode film,
   bonding an aluminum lid member, which has a sealant layer on one side and an adhesive layer on the other side, to the dissolution liquid-storing container via the sealant layer, and arranging the aluminum lid member within the flange outer diameter of the dissolution liquid-storing container, and
   bonding the aluminum lid member which is bonded to the dissolution liquid-storing container to the electrode film via the adhesive layer to cover the dissolution liquid passing hole.

### EFFECTS OF THE INVENTION

The iontophoresis device of the present invention has a structure capable of mixing the dissolution liquid and the drug by a simple operation and has the aluminum lid member, which is not exposed to the device surface, as the lid member of the dissolution liquid-storing container, so that there is no risk of leakage of electricity when applying current and its reliability and safety are excellent.

According to the method for producing the iontophoresis device of the present invention, the electrode film or the electrode film having the aluminum lid member can be mass-produced by using a material such as a rolled sheet or the like, and the iontophoresis device excelling in reliability and safety can be mass-produced easily.

### BEST MODE FOR CARRYING OUT THE INVENTION

Embodiments of the present invention are described below with reference to the drawings.

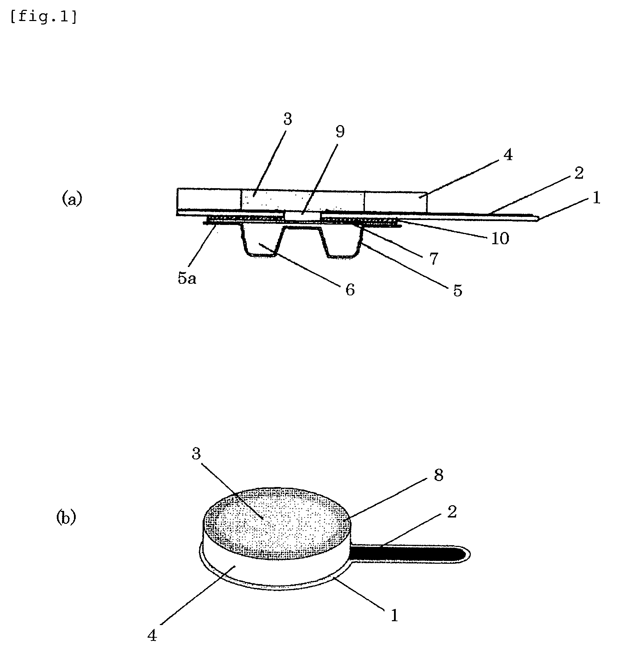

Fig. 1 is a diagram showing an embodiment of the iontophoresis device according to the present invention, where (a) is a sectional view and (b) is a perspective view. The iontophoresis device of this embodiment is mainly comprised of a base 1 formed of a polyethylene terephthalate (PET) film or the like, an electrode layer 2 formed on the base 1, a drug-loaded member 3 formed on the electrode layer 2, an expanded sheet 4 arranged along the circumference of the drug-loaded member 3, a dissolution liquid-storing container (dissolution liquid-storing blister container) 5 in which a dissolution liquid 6 is charged, and a lid member 7 which functions as a lid member of the dissolution liquid-storing container 5.

### (Electrode film)

As the base 1 of the electrode film, there are, for example, plastic films of polyethylene terephthalate (PET), polyimide, polyamide, polypropylene and the like, and the polyethylene terephthalate is particularly suitably used because it is excellent in insulation properties, heat resistance, machinability and the like. And, such films may be used as a single film or a composite film.

The electrode layer 2 is composed of a substantially circular part which becomes an electrode discharge portion and an extended part which becomes an electrode terminal portion. As a material for the electrode layer 2, for example, materials based on metal and nonmetal conductive materials such as silver, silver chloride, carbon, titanium, platinum, gold, aluminum, iron, nickel and a mixture of them can be used. A conductive paste based on such a material may also be used. By using such a conductive paste, the electrode layer can be formed by screen printing suitable for mass production.

For the electrode film having the electrode layer 2 formed on the base 1, a hole which becomes a dissolution liquid passing portion 9 to move the dissolution liquid 6 contained in the dissolution liquid-storing container 5 to the drug-loaded member 3 at the time of use is formed by punching fabrication.

### (Drug-loaded member)

The material for the drug-loaded member 3 is not limited to a particular one if it is a hydrophilicity based one and can absorb and hold a drug solution, and may be a cellulose fiber, a rayon fiber, a nylon fiber, a polyurethane foam, a polycarbonate foam, a polyvinyl alcohol foam, a polyester foam, a polyester nonwoven fabric, a polyester nonwoven fabric, cotton or a composite of them.

### (Expanded sheet)

The expanded sheet 4 has a function to prevent the drug solution from externally leaking out of the device when the drug-loaded member 3 is impregnated with the dissolution liquid. Therefore, it is required to be securely adhered to the electrode film. Since the device is used in a state attached to the skin, it is desired that the expanded sheet is a flexible soft foam having as a substrate various types of polymers such as polyurethane, polyethylene, polyvinyl chloride, polychloroprene, acrylic resin and polystyrene foams. And, since it is attached to the skin when used, it is desirable to have an adhesive 8 such as rubber-based, acrylic-based, silicone-based, polyvinyl-based, polyester-based, polyurethane-based or the like coated onto one side.

### (Sealing layer)

The drug-loaded member 3 and the expanded sheet 4 are bonded to the electrode film with a sealing layer (not shown), which is formed of an adhesive material or a heat-sealable material, provided in a prescribed region such as a peripheral portion of the electrode layer of the electrode film. The sealing layer is preferably heat sealable in view of an easy production process. Heat sealable materials are polydiene, polyacryl, polymethacryl, acrylamide, polyvinyl alcohol, polyethylene, polyvinyl ester, polystyrene, polycarbonate, polyester, polyurethane, polysiloxane, polyamide, polyacetal and polyacrylonitrile. Preferably, they are polydiene, polyacryl, polymethacryl, polyethylene, polyvinyl ester, polystyrene, polyester and polysiloxane. More preferably, they are polydiene, polyacryl, polymethacryl, polyester and polysiloxane but not limited to them. Adhesive materials are those having acryl or silicone as the but not limited to them.

### (Dissolution liquid-storing blister container)

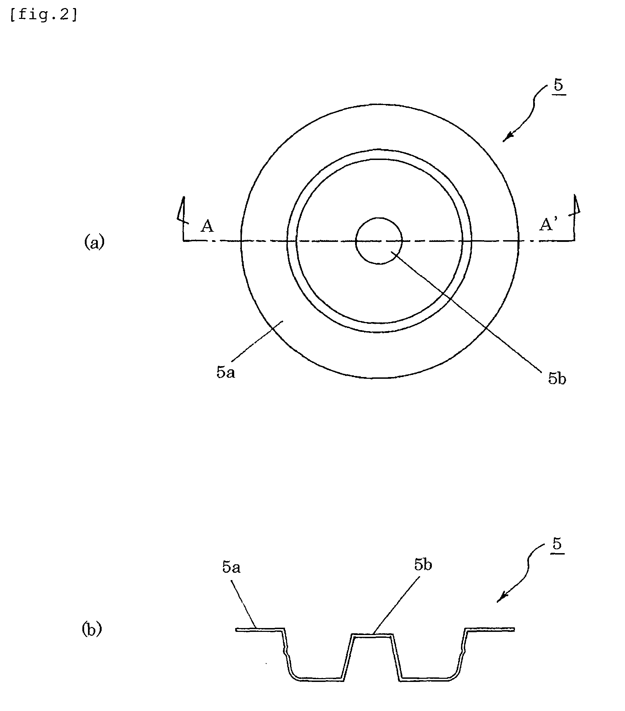

Fig. 2 is a diagram showing the dissolution liquid-storing blister container 5 of this embodiment, where (a) is a top view, and (b) is an A-A' sectional view of (a). As shown in the drawing, the dissolution liquid-storing blister container 5 has a flange portion 5a at the top of the outer circumference and a projected portion 5b at the center in the container.

For the dissolution liquid-storing blister container 5, it is desired to use a material having a high steam barrier property in order to prevent the dissolution liquid 6 from evaporating and decreasing its amount when the dissolution liquid 6 is charged and stored in the dissolution liquid-storing blister container 5. Such a material includes polyvinyl chloride, polyvinylidene chloride, polyethylene, polypropylene, polystyrene, polyamide, polymethylmethacrylate, polycarbonate, polyester, polyvinyl alcohol, polyvinyl acetate, thermoplastic elastomer, cyclic olefin polymer, cyclic olefin copolymer, a copolymer of ethylene with an organic carboxylic acid derivative having an ethylenically unsaturated bond, such as ethylene-methacrylate copolymer, ethylene-vinyl acetate copolymer, ethylene-acrylic acid copolymer and ethylene-vinyl acetate-methyl methacrylate copolymer, trifluoroethylene chloride resin, and the like. The dissolution liquid-storing blister container can be obtained by vacuum molding, compressed air molding or vacuum compressed air molding of a single layer or a sheet formed of laminated multilayers.

### (Lid member)

The lid member 7 is required to be excellent in a steam barrier property and a break-through characteristic such that permeation of moisture to the drug-loaded member 3 is prevented during storage and it is broken easily to mix the electrolyte 6 of the blister container 5 and the drug of the drug-loaded member 3 when used. Therefore, the present invention uses as the lid member 7 an aluminum lid member which has a sealant resin for sealing the blister container 5 coated to an aluminum foil. And, a half-cut line may be formed in the aluminum lid member to facilitate its break through.

It is important for the present invention that the aluminum lid member 7 is arranged within the outer diameter of the flange portion 5a of the blister container 5. In other words, since the iontophoresis system essentially needs application of current when it is used, if aluminum is used for the lid member, it is necessary to prevent a leakage of electricity from the aluminum lid member. But, when a lid member having the same shape (more preferably smaller than the flange) as the flange of the blister container is arranged within the outer diameter of the flange, the lid member is exposed to the outside of the device, and a leakage of electricity can be prevented effectively.

Since the aluminum lid member 7 is not directly adhered to a plastic film of PET or the like which is suitably used as the base 1 of the electrode film, the present invention is desirable that the electrode film and the aluminum lid member are bonded via an adhesive layer 10. The adhesive to be used for the adhesive layer 10 is not particularly limited, but a rubber-based, acrylic-based, silicone-based, polyvinyl-based, polyester-based or polyurethane-based adhesive is suitably used in view of safety and elimination of disadvantages involved at the time of moistening.

To bond the electrode film and the aluminum lid member via the adhesive layer, the aluminum lid member may be sealed with the blister container and then bonded to the adhesive layer formed on the electrode film, or the electrode film and the aluminum lid member may be laminated in advance before the blister container is sealed. It is desirable to laminate the electrode film and the aluminum lid member in advance because a gap between the aluminum lid member and the adhesive layer is eliminated and a loss of the dissolution liquid due to capillary action can be prevented effectively.

At the time of using the iontophoresis device configured as described above, the device is applied to the skin with the adhesive 8 provided on the top surface of the expanded sheet 4, the dissolution liquid-storing blister container 5 is pushed by a finger to break the resin lid member 7 by the projected portion 5b formed in the blister container to migrate the dissolution liquid 6 to the drug-loaded member 3 through the dissolution liquid passing portion 9 to mix with the drug. And, a current is passed to the electrode 2 to ionize the drug solution to introduce it into the body through the skin.

Embodiments of a method for producing an iontophoresis device of the present invention are described below.

### (Embodiment 1)

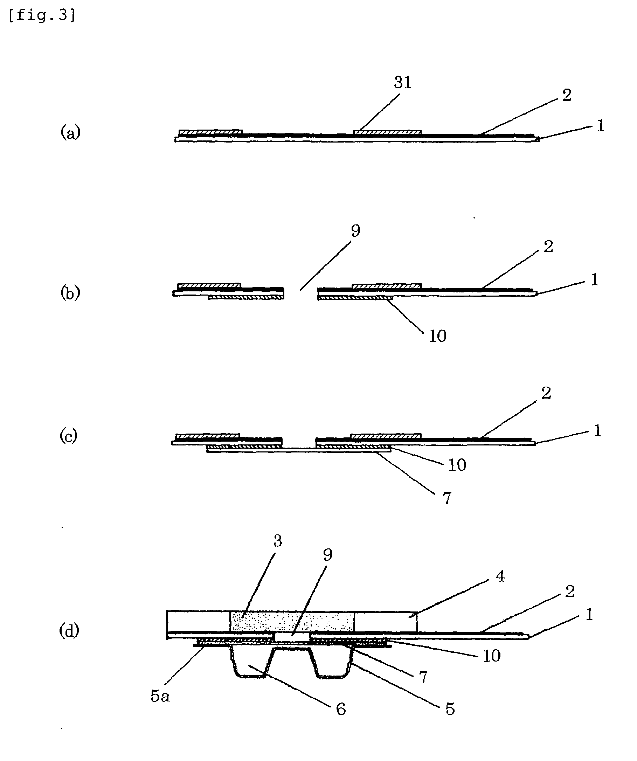

This embodiment relates to a method for producing the iontophoresis device configured as exemplified in Fig. 1. Fig. 3 shows a diagram of its production process.

The production method of this embodiment has a step of forming an adhesive layer on only a part of an electrode film to which a dissolution liquid-storing container is bonded, a step of forming a dissolution liquid passing hole in an inside region of the electrode film to which the adhesive layer was formed, a step of bonding an aluminum lid member having a sealant layer on its one side to an adhesive layer, and a step of bonding the dissolution liquid-storing container via the sealant layer of the aluminum lid member and arranging the aluminum lid member within the outer diameter of the flange of the dissolution liquid-storing container.

First, an electrode layer 2 is formed on a base 1 of a PET film or the like, and a sealing layer 31 is formed on a prescribed region such as a peripheral portion of the electrode layer (Fig. 3(a)).

Then, an adhesive layer 10 is coated on the bonded portion of the base 1 with the dissolution liquid-storing blister container, and a hole which becomes a dissolution liquid passing portion 9 is formed in an inside region of the formed adhesive layer 10 by punching fabrication to obtain the electrode film (Fig. 3(b)).

As the adhesive coating method described above, there can be used various coating methods such as gravure coating, reverse coating, lip coating, die coating, comma coating, knife coating, screen printing, calender coating, hot melt-coating and the like.

Then, an aluminum lid member 7 which has a sealant layer formed on its one side in advance is laminated on the adhesive layer-formed side of the electrode film, a half-cut line is formed in the aluminum lid member along the bonded portion with the blister, and a portion of the aluminum lid member which is not in contact with the adhesive layer is removed. Thus, the lid member-laminated electrode film with the aluminum lid member 7 bonded to only the portion forming the adhesive layer 10 can be obtained (Fig. 3(c)).

According to the present invention, the aluminum lid member 7 is arranged within only the flange outer diameter of the dissolution liquid-storing blister container, so that the adhesive layer 10 is not formed on the entire surface of the device but only the bonded portion. Therefore, in a case where the adhesive layer is formed on the electrode film side, the adhesive layer is formed by the method of partial coating on the bonded portion only as in this embodiment or a method (described in Embodiment 2) of laminating a mask film which is coated on the entire surface and has a hole formed in the bonded portion only. Subsequently, lamination with the aluminum lid member causes bonding with only the portion where the adhesive layer is formed, so that when a half-cut line is formed in the aluminum lid member along the bonded portion, the aluminum lid member remains on only the bonded portion as described above, and the portion of the lid member which is not in contact with the adhesive can be removed easily.

A drug-loaded member 3 and an expanded sheet 4 are provided on the sealing layer 31 which is formed on the electrode side of the lid member-laminated electrode film and bonded by heat sealing.

Then, a flange surface 5a of a dissolution liquid-storing blister container 5, in which a dissolution liquid 6 is charged, is bonded to the lid member surface of the lid member-laminated electrode film by sealing to complete the iontophoresis device of the present invention that the aluminum lid member 7 is arranged within the flange outer diameter of the blister container 5 (Fig. 3(d)). The method of sealing the flange surface 5a of the dissolution liquid-storing blister container 5 to the aluminum lid member surface is not particularly limited, but various types of methods such as heat sealing, impulse sealing, ultrasonic sealing, high-frequency sealing and the like can be used.

### (Embodiment 2)

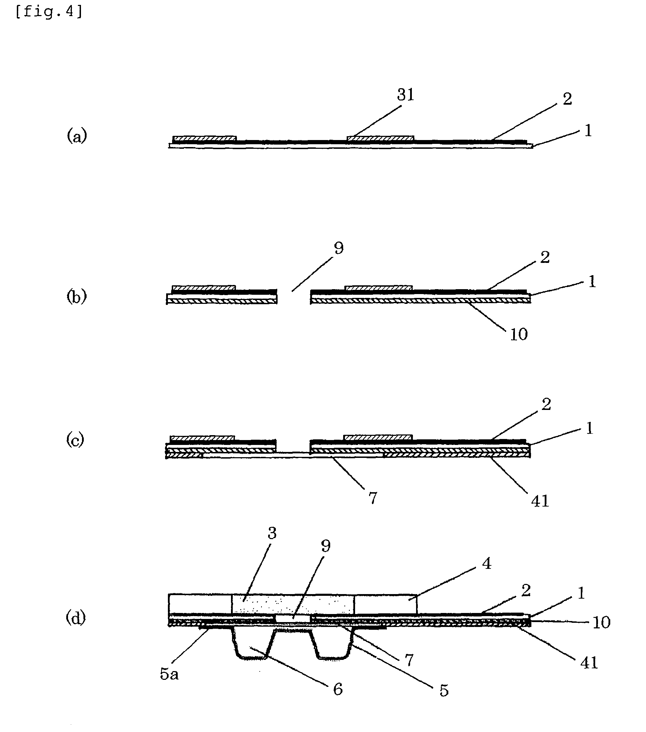

In Embodiment 1, the adhesive layer was formed on only the portion of bonding the electrode film to the dissolution liquid-storing container, but Embodiment 2 is an example of forming the adhesive layer on the entire surface of a side of the electrode film to which the dissolution liquid-storing container is bonded, and Fig. 4 is a diagram showing its production process.

The production method of this embodiment has a step of forming an adhesive layer on the entire surface of a side of an electrode film to which a dissolution liquid-storing container is bonded, a step of forming a dissolution liquid passing hole in the electrode film, a step of laminating a coating film having an opening larger than a dissolution liquid passing hole and an aluminum lid member having a sealant layer on one side surface to an adhesive layer-formed side of the electrode film to bond the aluminum lid member to the adhesive layer at the opening portion, and a step of bonding the dissolution liquid-storing container via the sealant layer of the aluminum lid member to arrange the aluminum lid member within the outer diameter of the flange of the dissolution liquid-storing container.

First, an electrode layer 2 is formed on a base 1 such as a PET film, and a sealing layer 31 is formed on a prescribed region such as a peripheral portion of the electrode layer (Fig. 4(a)).

Then, an adhesive layer 10 is coated on the entire surface of the non-printed side of the base 1 and a hole which becomes a dissolution liquid passing portion 9 is formed by punching fabrication to obtain the electrode film (Fig. 4(b)).

Then, a coating film 41 having an opening which is larger than the dissolution liquid passing hole 9 and smaller than the flange outer diameter of a dissolution liquid-storing blister container 5 and an aluminum lid member 7 which has the sealant layer previously formed on one side surface are laminated on the adhesive layer-formed side of the electrode film. A half-cut line is formed in the aluminum lid member along the bonded portion with the blister, and a portion of the aluminum lid member which is not in contact with the adhesive layer is removed. Thus, the lid member-laminated electrode film which has the aluminum lid member 7 bonded to the adhesive layer 10 at the opening portion of the coating film 41 can be obtained (Fig. 4(c)).

Similar to Embodiment 1, a drug-loaded member 3, an expanded sheet 4 and the dissolution liquid-storing blister container 5 are fitted to the lid member-laminated electrode film to complete the iontophoresis device of the present invention that the aluminum lid member 7 is arranged within the flange outer diameter of the blister container 5 (Fig. 4(d)).

### (Embodiment 3)

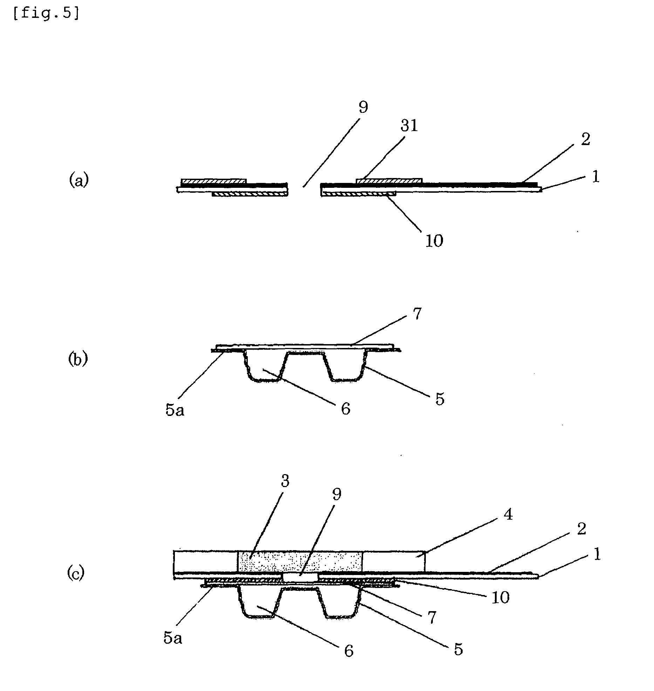

In Embodiments 1 and 2, the dissolution liquid-storing container was fitted to the electrode film having the lid member previously laminated, but Embodiment 3 is an example of forming the electrode film in a state that the lid member is not laminated and fitting to it a blister container having the lid member applied in advance, and Fig. 5 is a diagram showing its production process.

The production method of this embodiment has a step of forming an adhesive layer on only a part of an electrode film to which a dissolution liquid-storing container is bonded, a step of forming a dissolution liquid passing hole in an inside region of the electrode film to which the adhesive layer was formed, a step of bonding an aluminum lid member within the flange outer diameter of the dissolution liquid-storing container, and a step of bonding the aluminum lid member, which is bonded to the dissolution liquid-storing container, to the adhesive layer.

Similar to Embodiment 1, an electrode layer 2 is formed on a base 1 of a PET film or the like, a sealing layer 31 is formed on a prescribed region such as a peripheral portion of the electrode layer, an adhesive layer 10 is coated on a bonded portion of the base 1 with a dissolution liquid-storing blister container, and a hole which becomes a dissolution liquid passing portion 9 is formed by punching fabrication in a region of the inner side where the adhesive layer 10 is formed to obtain an electrode film (Fig. 5(a)).

An aluminum lid member 7 which has a sealant layer formed on its one side surface in advance is laminated for sealing on a flange 5a of a dissolution liquid-storing blister container 5, in which a dissolution liquid 6 is charged, to bond the aluminum lid member 7 within the flange outer diameter of the blister container 5 (Fig. 5(b)).

A drug-loaded member 3 and an expanded sheet 4 are provided on the sealing layer 31 which is formed on the electrode side of the electrode film and bonded by heat sealing.

Then, the blister container 5 to which the aluminum lid member 7 was adhered is bonded to the adhesive layer-formed side of the electrode film to complete the iontophoresis device (Fig. 5(c)).

In this embodiment, the adhesive layer is formed on only a portion of the electrode film to which the dissolution liquid-storing container is bonded, but similar to Embodiment 2, the adhesive layer is also applied formed on the entire surface on the side of the electrode film to which the dissolution liquid-storing container is bonded.

### (Embodiment 4)

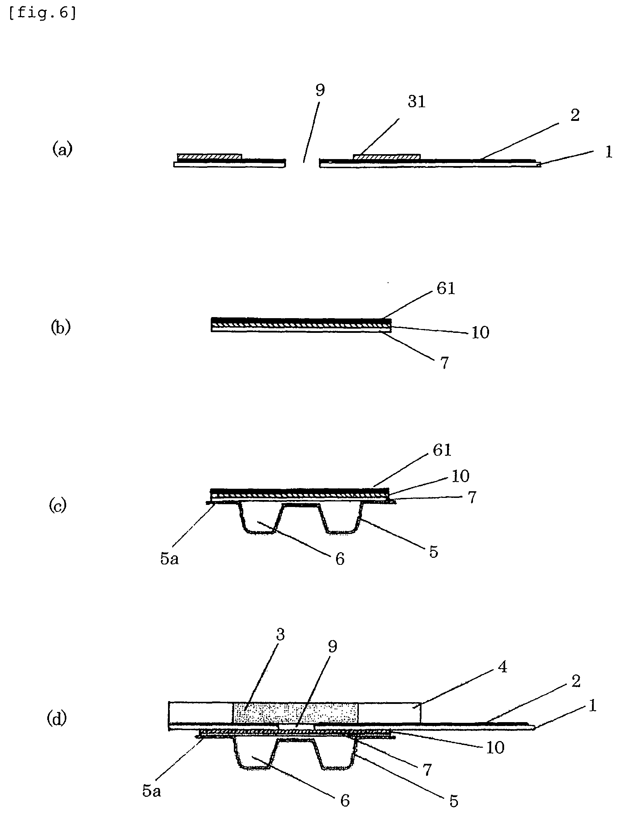

In the respective embodiments described above, the adhesive layer was formed on the electrode film side, but Embodiment 4 is an example of forming the adhesive layer on the lid member, and Fig. 6 is a diagram showing its production process.

The production method of this embodiment has a step of forming a dissolution liquid passing hole in an electrode film, a step of bonding an aluminum lid member, which has a sealant layer on one side and an adhesive layer on the other side, to a dissolution liquid-storing container via the sealant layer to arrange the aluminum lid member within the flange outer diameter of the dissolution liquid-storing container, and a step of bonding the aluminum lid member, which is bonded to the dissolution liquid-storing container, to the electrode film via the adhesive layer to cover the dissolution liquid passing hole.

First, an electrode layer 2 is formed on a base 1 of a PET film or the like, a sealing layer 31 is formed on a prescribed region such as a peripheral portion of the electrode layer, and a hole which becomes a dissolution liquid passing portion 9 is formed by punching fabrication to obtain an electrode film (Fig. 6(a)).

An adhesive layer 10 is formed on a release surface of a release film 61, and the aluminum side of an aluminum lid member 7 having a sealant layer previously formed on one side surface is laminated on the adhesive layer-formed side to obtain an adhesive layer-formed aluminum lid member (Fig. 6(b)).

Then, the adhesive layer-formed aluminum lid member is bonded to a flange 5a of a dissolution liquid-storing blister container 5, in which a dissolution liquid 6 is charged via the sealant layer, to arrange an aluminum lid member 7 within the flange outer diameter of the blister container 5 (Fig. 6(c)).

A drug-loaded member 3 and an expanded sheet 4 are positioned on the sealing layer 31 formed on the electrode side of the electrode film, and they were bonded by heat sealing.

A release film 61 of the adhesive layer-formed aluminum lid member applied to the blister container 5 is removed to bond to the electrode film via the adhesive layer 10 to cover the dissolution liquid passing hole 9 with the aluminum lid member 7 to complete the iontophoresis device (Fig. 6(d)).

In the respective embodiments described above, since the dissolution liquid passing hole 9 is formed after the adhesive layer 10 is formed on the electrode film side, there is no adhesive layer 10 on the region of the dissolution liquid passing hole 9. Therefore, the adhesive layer 10 does not affect on piercing strength when the aluminum lid member 7 is broken at the time of use, and the dissolution liquid and the drug can be mixed easily.

In a case where the adhesive layer is coated on the lid member side as in this embodiment, and especially when an adhesive which considerably affects on piercing strength is used, the region of the dissolution liquid passing hole 9 is formed to have a single layer structure of the lid member only, so that it is desirable to coat the adhesive by partial coating.

As described above, the iontophoresis device of the present invention has the aluminum lid member, which is used as the lid member of the blister container, bonded to the electrode film to integrate the dissolution liquid-storing blister container with the electrode film, and since the aluminum lid member is not exposed to the device surface, the realized iontophoresis device has a structure capable of mixing the dissolution liquid and the drug by a simple operation. And, there is no risk of leakage of electricity when applying current and the iontophoresis device has excellent reliability and safety.

According to the method for producing the iontophoresis device of the present invention, the electrode film (or the aluminum lid member-formed electrode film) can be mass-produced by using a material such as a rolled sheet or the like, and especially according to the method of forming the adhesive layer on the entire surface of the electrode film as in Embodiment 2, substantially the entire production process of the aluminum lid member-formed electrode film can be performed continuously, and the iontophoresis device excelling in reliability and safety can be mass-produced easily.

### EXAMPLES

Specific examples of the present invention are described below but the present invention is not limited to them.

### [Example 1]

Example 1 is an example of producing an iontophoresis device by the production process shown in Fig. 3.

### (Formation of lid member-laminated electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. A sealing layer 31 having a thickness of 10 µm was formed by screen printing an ink composed of a thermoplastic saturated copolymer polyester resin on a prescribed region such as a peripheral portion of the electrode layer 2 (Fig. 3(a)). Then, an adhesive layer 10 was formed on a non-printed surface of the base by partial coating of an acrylic-based adhesive (trade name "SK-Dyne" produced by Soken Chemical & Engineering Co., Ltd.) on a portion (a circular region 1 mm inside from the flange outer diameter of a blister) bonded to the blister, and a hole which became a dissolution liquid passing portion 9 was formed by punching fabrication to obtain an electrode film (Fig. 3(b)).

An aluminum lid member 7, which had a sealant resin (maleinated polypropylene) coated on a hard aluminum foil having a thickness of 20 µm, was laminated on the adhesive layer-formed side of the electrode film, a half-cut line was formed in the aluminum lid member along the bonded portion with the blister, and a portion of the aluminum lid member which was not in contact with the adhesive layer was removed to obtain a lid member-laminated electrode film (Fig. 3(c)).

### (Formation of dissolution liquid-storing blister container)

A dissolution liquid-storing blister container 5 having a projection at the center portion was obtained by vacuum molding of a resin sheet (trade name "SUMILITE" produced by Sumitomo Bakelite Co. Ltd.) for PTP (Press Through Package). The flange portion of the blister container had an outer diameter of 30 mm and an inner diameter of 20 mm.

### (Assembling of device)

An expanded olefin sheet 4 (trade name "Volara" produced by SEKISUI CHEMICAL CO., LTD.) and a drug-loaded nonwoven fabric 3 were arranged on the sealing layer 31 formed on the electrode side of the lid member-laminated electrode film and bonded by heat sealing. Subsequently, a dissolution liquid 6 was charged into the dissolution liquid-storing blister container 5, a flange surface of the blister container was bonded to the lid member surface of the lid member-laminated electrode film by impulse sealing to complete the iontophoresis device.

The iontophoresis device of this example has the aluminum lid member (an outer diameter of 28 mm) arranged 1 mm inside from the flange 5a of the blister container having an outer diameter 30 mm, and the aluminum lid member is not exposed to the device surface. Therefore, the iontophoresis device obtained is free from a possibility of causing a leakage of electricity when current is applied and excellent in reliability and safety.

Since the production method of this example forms the lid member-laminated electrode film by performing partial coating of the adhesive on only the bonded portion of the electrode film with the blister and laminating the aluminum lid member on it, the aluminum lid member having a prescribed size can be arranged at a prescribed position easily, and a gap is not formed between the electrode film and the lid member, and there is no risk of leakage of the dissolution liquid between the electrode film and the lid member at the time of use.

### [Example 2]

In Example 2, an iontophoresis device was produced in the same manner as in Example 1 except that a procedure of producing the lid member-laminated electrode film was different. The production of the lid member-laminated electrode film in Example 2 is described below.

### (Formation of lid member-laminated electrode film)

An adhesive layer 10 was formed on a base 1 of a biaxially stretched polyethylene terephthalate (PET) film having a thickness of 75 µm on a portion (a circular region 1 mm inside from the flange outer diameter of the blister) bonded to the blister by partial coating of an acrylic-based adhesive (trade name "SK-Dyne" produced by Soken Chemical & Engineering Co., Ltd.), and a release paper was laminated on the adhesive layer 10. Conductive carbon and a conductive silver chloride paste ink were screen printed on the surface opposite to the adhesive-coated surface to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2. Then, a hole which became a dissolution liquid passing portion 9 was formed by punching fabrication to obtain an electrode film (Fig. 3(b)).

The release paper was removed from the electrode film, an aluminum lid member 7, which had a sealant resin (maleinated polypropylene) coated on the hard aluminum foil having a thickness of 20 µm, was laminated on the adhesive layer-formed side, a half cut line was formed in the aluminum lid member along the bonded portion with the blister, and a portion of the aluminum lid member, which was not in contact with the adhesive layer, was removed to obtain the lid member-laminated electrode film (Fig. 3(c)).

An iontophoresis device was completed by the same manner as in Example 1.

Since the aluminum lid member of this example is not exposed to the device surface either, the iontophoresis device obtained is free from a possibility of causing a leakage of electricity when current is applied and excellent in reliability and safety. And, the aluminum lid member having a prescribed size can be arranged at a prescribed position easily, a gap is not formed between the electrode film and the lid member, and there is no risk of leakage of the dissolution liquid between the electrode film and the lid member at the time of use.

### [Example 3]

Example 3 is an example of producing an iontophoresis device by the production process shown in Fig. 4.

### (Formation of lid member-laminated electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. A sealing layer 31 having a thickness of 10 µm was formed by screen printing an ink composed of a thermoplastic saturated copolymer polyester resin on a prescribed region such as a peripheral portion of the electrode layer 2 (Fig. 4(a)). Then, an adhesive layer 10 was formed on a non-printed surface of the base by comma coating of an acrylic-based adhesive (trade name "SK-Dyne" produced by Soken Chemical & Engineering Co., Ltd.), and a hole which became a dissolution liquid passing portion 9 was formed by punching fabrication (Fig. 4(b)).

Then, a PET film 41 having a thickness of 12 µm and fabricated to have a hole having a diameter of 28 mm along the bonded portion with the blister and an aluminum lid member 7 which had a sealant resin (maleinated polypropylene) coated on a hard aluminum foil having a thickness of 20 µm were laminated on the adhesive layer-formed side, a half cut line was formed in the aluminum lid member along the bonded portion with the blister, and a portion of the aluminum lid member which was not in contact with the adhesive layer was removed to obtain a lid member-laminated electrode film (Fig. 4(c)).

An iontophoresis device was completed by the same manner as in Example 1.

Since the aluminum lid member of this example is not exposed to the device surface either, the iontophoresis device obtained is free from a possibility of causing a leakage of electricity when current is applied and excellent in reliability and safety. The aluminum lid member having a prescribed size can be arranged at a prescribed position easily, a gap is not generated between the electrode film and the lid member, and there is no risk of leakage of the dissolution liquid between the electrode film and the lid member at the time of use. And, an adhesive is coated on the entire surface of one side surface of the electrode film, a mask film which has a hole formed in the bonded portion with the blister is laminated, and the aluminum lid member is laminated on it to form the lid member-laminated electrode film, so that the lid member-laminated electrode film can be mass-produced extremely efficiently by using a rolled member.

### [Example 4]

In Example 4, an iontophoresis device was produced in the same manner as in Example 3 except that a procedure of producing the lid member-laminated electrode film was different. The production of the lid member-laminated electrode film in Example 4 is described below.

### (Formation of lid member-laminated electrode film)

An adhesive layer 10 was formed on the entire surface of one side of a base 1 of a biaxially stretched polyethylene terephthalate (PET) film having a thickness of 75 µm by comma coating an acrylic-based adhesive (trade name "SK-Dyne" produced by Soken Chemical & Engineering Co. , Ltd.), and a release paper was laminated on the adhesive layer 10. Conductive carbon and a conductive silver chloride paste ink were screen printed on the surface opposite to the adhesive-coated surface to form an electrode layer 2 having a thickness of 30 µm. A sealing layer 31 having a thickness of 10 µm was formed by screen printing an ink composed of a thermoplastic saturated copolymer polyester resin on a prescribed region such as a periphery of the electrode layer 2. Then, a hole which became a dissolution liquid passing portion 9 was formed by punching fabrication (Fig. 4(b)).

After the release paper was removed, a PET film 41 having a thickness of 12 µm and fabricated to have a hole having a diameter of 28 mm and an aluminum lid member 7 which had a sealant resin (maleinated polypropylene) coated on a hard aluminum foil having a thickness of 20 µm were laminated on the adhesive layer-formed side along the bonded portion with the blister, a half cut line was formed in the aluminum lid member along the bonded portion with the blister, and a portion of the aluminum lid member which was not in contact with the adhesive layer was removed to obtain a lid member-laminated electrode film (Fig. 4(c)).

The iontophoresis device was completed in the same manner as in Example 1 as described below.

Since the aluminum lid member of this example is not exposed to the device surface either, the iontophoresis device obtained is free from a possibility of causing a leakage of electricity when current is applied and excellent in reliability and safety. The aluminum lid member having a prescribed size can be arranged at the prescribed position easily and a gap is not formed between the electrode film and the lid member, and there is no risk of leakage of the dissolution liquid between the electrode film and the lid member at the time of use. And, the adhesive is coated on the entire surface of one side surface of the electrode film, a mask film which has a hole formed in only the bonded portion with the blister is laminated, and the aluminum lid member is laminated on it to form the lid member-laminated electrode film, so that the lid member-laminated electrode film can be mass-produced extremely efficiently by using a rolled member.

### [Example 5]

Example 5 is an example of producing an iontophoresis device by the production process shown in Fig. 5.

### (Formation of electrode film)

Similar to Example 1, an electrode layer 2 was formed on a base 1 of a PET film, a sealing layer 31 was formed on a prescribed region such as a periphery or the like of the electrode layer, an adhesive layer 10 was coated on a non-printed side of the base 1 at the bonded portion with the dissolution liquid-storing blister container, and a hole which became a dissolution liquid passing portion 9 was formed by punching fabrication in a region of the inner side where the adhesive layer 10 is formed to obtain an electrode film (Fig. 5(a)).

### (Formation of dissolution liquid-storing blister container)

A dissolution liquid-storing blister container 5 having a projection at the center portion was obtained by vacuum molding of a resin sheet (trade name "SUMILITE" produced by Sumitomo Bakelite Co. Ltd.) for PTP. The flange portion of the blister container had an outer diameter of 30 mm and an inner diameter of 20 mm.

A dissolution liquid 6 was charged into the dissolution liquid-storing blister container 5, an aluminum lid member 7 (outer diameter 28 mm), which had a sealant resin (maleinated polypropylene) coated on a hard aluminum foil having a thickness of 20 µm, was laminated 1 mm inside from a flange 5a of the blister container and sealed by heat sealing (Fig. 5(b)).

### (Assembling of device)

An expanded olefin sheet 4 (trade name "Volara" produced by SEKISUI CHEMICAL CO., LTD.) and a drug-loaded nonwoven fabric 3 were arranged on the sealing layer 31 formed on the electrode side of the electrode film and bonded by heat sealing. Subsequently, the blister container 5 to which the aluminum lid member 7 was applied was bonded to the adhesive layer-formed side of the electrode film to complete an iontophoresis device (Fig. 5(c)).

Since the aluminum lid member of this example is not exposed to the device surface either, the iontophoresis device obtained is free from a possibility of causing a leakage of electricity when current is applied and excellent in reliability and safety.

### [Example 6]

Example 6 is an example of producing an iontophoresis device by the production method shown in Fig. 6.

### (Formation of electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2. Then, a hole which became a dissolution liquid passing portion 9 was formed by punching fabrication to obtain an electrode film (Fig. 6(a)).

### (Formation of adhesive layer-formed aluminum lid member)

An adhesive layer 10 was formed on a release surface of a release film 61 by comma coating an acrylic-based adhesive (trade name "SK-Dyne" produced by Soken Chemical & Engineering Co. , Ltd.), and the aluminum side of an aluminum lid member 7 (outer diameter 28 mm) having a sealant resin (maleinated polypropylene) coated on a hard aluminum foil having a thickness of 20 µm was laminated on the adhesive layer-formed side (Fig. 6(b)).

### (Formation of dissolution liquid-storing blister container)

A dissolution liquid-storing blister container 5 having a projection at the center portion was obtained by vacuum molding of a resin sheet (trade name "SUMILITE" produced by Sumitomo Bakelite Co. Ltd.) for PTP. The flange portion of the blister container had an outer diameter of 30 mm and an inner diameter of 20 mm.

A dissolution liquid 6 was charged into the dissolution liquid-storing blister container 5, and the sealant resin side of the adhesive layer-formed aluminum lid member was laminated 1 mm inside from a flange 5a of the blister container and sealed by heat sealing (Fig. 6(c)).

### (Assembling of device)

An expanded olefin sheet 4 (trade name "Volara" produced by SEKISUI CHEMICAL CO., LTD.) and a drug-loaded nonwoven fabric 3 were positioned on the adhesive sealing layer 31 which was formed on the electrode side of the electrode film and bonded mutually by heat sealing. Subsequently, the release film 61 was removed from the adhesive layer-formed aluminum lid member applied to the blister container to bond to the electrode film to complete an iontophoresis device (Fig. 6(d)).

Since the aluminum lid member of this example is not exposed to the device surface either, the iontophoresis device obtained is free from a possibility of causing a leakage of electricity when current is applied and excellent in reliability and safety.

### [Example 7]

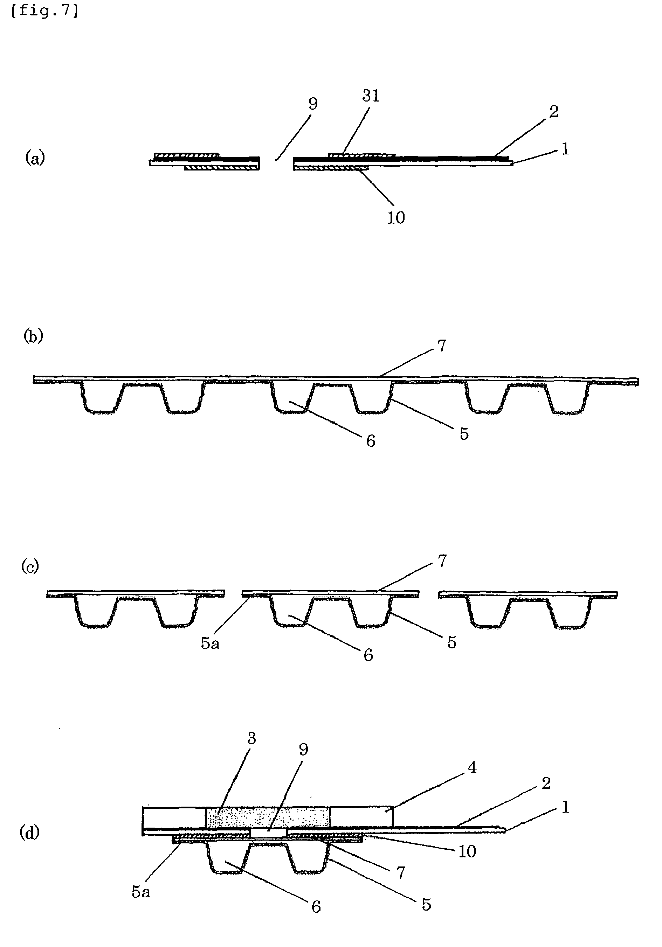

Example 7 is an example of producing an iontophoresis device by the production process of Fig. 7.

### (Formation of electrode film)

Similar to Example 1, an electrode layer 2 was formed on a base 1 of a PET film, a sealing layer 31 was formed on a prescribed region such as a periphery of the electrode layer, an adhesive layer 10 was coated on a non-printed side of the base 1 at the bonded portion with the dissolution liquid-storing blister container, and a hole which became a dissolution liquid passing portion 9 was formed by punching fabrication in a region of the inner side where the adhesive layer 10 was formed to obtain an electrode film (Fig. 7(a)).

### (Formation of dissolution liquid-storing blister container)

A plurality of the dissolution liquid-storing blister containers having a projection at the center portion were produced by vacuum molding of a resin sheet (trade name "SUMILITE" produced by Sumitomo Bakelite Co. Ltd.) for PTP at a time, a dissolution liquid 6 was charged, and an aluminum lid member 7, which had a sealant resin (maleinated polypropylene) coated on a hard aluminum foil as the lid member, was sealed by heat sealing (Fig. 7(b)). Then, a plurality of the dissolution liquid-storing blister containers 5 were obtained by cutting at the same time (Fig. 7(c)). The dissolution liquid-storing blister containers 5 of Example 7 obtained as described above have the same aluminum lid member as the outer diameter of a flange 5a arranged as the lid member.

### (Assembling of device)

An expanded olefin sheet 4 (trade name "Volara" produced by SEKISUI CHEMICAL CO., LTD.) and a drug-loaded nonwoven fabric 3 were positioned on the sealing layer 31 which was formed on the electrode side of the electrode film and bonded mutually by heat sealing. Subsequently, the blister container to which the aluminum lid member 7 was applied was bonded to the adhesive layer-formed side of the electrode film to complete an iontophoresis device (Fig. 7(d)).

Since the aluminum lid member of this example is not exposed to the device surface either, the iontophoresis device obtained is free from a possibility of causing a leakage of electricity when current is applied and excellent in reliability and safety.
In this example, a plurality of the blister containers sealed with the aluminum lid can be produced in bulk, so that mass production can be made in a larger scale.

### BRIEF DESCRIPTION OF THE DRAWINGS

[Fig. 1] Diagram showing an embodiment of an iontophoresis device according to the present invention, where (a) is a sectional view and (b) is a perspective view.
[Fig. 2] Diagram showing a dissolution liquid-storing blister container according to an embodiment of an iontophoresis device according to the present invention, where (a) is a top view and (b) is a A-A' sectional view of (a).
[Fig. 3] Diagram showing a production process for description of an example of a method for producing an iontophoresis device of the present invention.
[Fig. 4] Diagram showing a production process for description of another example of a method for producing an iontophoresis device of the present invention.
[Fig. 5] Diagram showing a production process for description of another example of a method for producing an iontophoresis device of the present invention.
[Fig. 6] Diagram showing a production process for description of another example of a method for producing an iontophoresis device of the present invention.
[Fig. 7] Diagram showing a production process for description of another example of a method for producing an iontophoresis device of the present invention.

### EXPLANATION OF REFERENCE NUMERALS

1: Base of electrode film
2: Electrode layer
3: Drug-loaded member
4: Expanded sheet
5: Dissolution liquid-storing container (Dissolution liquid-storing blister container)
5a: Flange
5b: Projected portion (Projection)
6: Dissolution liquid
7: Resin lid member
8: Adhesive
9: Dissolution liquid passing portion
10: Adhesive layer
31: Sealing layer
41: Coating film (Mask film)
61: Release film

## Claims

1. An iontophoresis device, comprising at least an electrode film having an electrode layer formed on a base, a drug-loaded member arranged on the electrode-layer-forming side of the electrode film, and a dissolution liquid-storing container arranged on the non-electrode-layer-forming side of the electrode film, wherein:
the electrode film is provided with a dissolution liquid passing hole, a flange of the dissolution liquid-storing container is bonded to the electrode film via an aluminum lid member which covers the dissolution liquid passing hole, and the aluminum lid member is arranged within the flange outer diameter of the dissolution liquid-storing container.

2. The iontophoresis device according to claim 1, wherein the aluminum lid member and the electrode film are bonded via an adhesive layer.

3. The iontophoresis device according to claim 1 or 2, wherein the surface of the aluminum lid member positioned at the dissolution liquid passing hole does not have the adhesive layer.

4. The iontophoresis device according to any one of claims 1 to 3, wherein the aluminum lid member and the dissolution liquid-storing container are bonded via a sealant layer.

5. A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
forming an adhesive layer on only a part of the electrode film to which the dissolution liquid-storing container is bonded,
forming a dissolution liquid passing hole in a region of the inner side of the electrode film where the adhesive layer is formed,
bonding an aluminum lid member having a sealant layer on one side surface to the adhesive layer, and
bonding the dissolution liquid-storing container via the sealant layer of the aluminum lid member, and arranging the aluminum lid member within the flange outer diameter of the dissolution liquid-storing container.

6. A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
forming an adhesive layer on the entire surface of the side of the electrode film to which the dissolution liquid-storing container is bonded,
forming a dissolution liquid passing hole in the electrode film,
laminating a coating film having an opening larger than the dissolution liquid passing hole and an aluminum lid member having a sealant layer on one side surface on the adhesive layer-formed side of the electrode film, and bonding the aluminum lid member to the adhesive layer at the opening portion, and
bonding the dissolution liquid-storing container via the sealant layer of the aluminum lid member, and arranging the aluminum lid member within the flange outer diameter of the dissolution liquid-storing container.

7. A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
forming an adhesive layer on only a part of the electrode film to which the dissolution liquid-storing container is bonded,
forming a dissolution liquid passing hole in a region of the inner side of the electrode film where the adhesive layer is formed,
bonding an aluminum lid member within the flange outer diameter of the dissolution liquid-storing container, and
bonding the aluminum lid member which is bonded to the dissolution liquid-storing container to the adhesive layer.

8. A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
forming an adhesive layer on the entire surface of the side of the electrode film to which the dissolution liquid-storing container is bonded,
forming a dissolution liquid passing hole in the electrode film,
bonding a coating film having an opening larger than the dissolution liquid passing hole to the adhesive layer-formed side of the electrode film,
bonding an aluminum lid member within the flange outer diameter of the dissolution liquid-storing container, and
bonding the aluminum lid member which is bonded to the dissolution liquid-storing container to the adhesive layer at the opening portion of the coating film.

9. A method for producing an iontophoresis device which comprises at least an electrode film having an electrode layer formed on a base, a drug-loaded member arranged on the electrode-layer-forming side of the electrode film, and a dissolution liquid-storing container arranged on the non-electrode-layer-forming side of the electrode film, comprising:
forming a dissolution liquid passing hole in the electrode film,
bonding an aluminum lid member, which has a sealant layer on one side and an adhesive layer on the other side, to the dissolution liquid-storing container via the sealant layer, and arranging the aluminum lid member within the flange outer diameter of the dissolution liquid-storing container, and
bonding the aluminum lid member which is bonded to the dissolution liquid-storing container to the electrode film via the adhesive layer to cover the dissolution liquid passing hole.
